# FASCICULE DE BREVET EUROPEEN

(11) **EP 0 774 504 B1**
(45) Date de publication et mention de la délivrance du brevet: **22.01.2003**
(21) Numéro de dépôt: 96490044.3
(22) Date de dépôt: 18.11.1996
(51) Int. Cl.: C11D 3/00, C11D 3/386

(54) **Composition pour nettoyer et/ou désinfecter des instruments à usage médical**
Zusammensetzung zur Reinigung und/oder Desinfektion von medizinischen Geräten
Composition for cleaning and/or disinfecting medical instruments

(30) Priorité: 17.11.1995 FR 9513887
(43) Date de publication de la demande: 21.05.1997
(73) Titulaire: Laboratoires ANIOS S.A., 59260 Lille-Hellemmes (Nord) (FR)
(72) Inventeur: Letarte, Bertrand, 59260 Lille-Hellemmes (FR)
(74) Mandataire: Duthoit, Michel

(56) Documents cités:
- EP-A- 0 099 209
- EP-A- 0 206 418
- EP-A- 0 290 223
- EP-A- 0 342 499
- EP-A- 0 481 663
- DE-A- 2 130 833
- DE-A- 4 343 128
- FR-A- 2 273 556
- US-A- 4 456 544
- DATABASE WPI Section Ch, Week 8915 Derwent Publications Ltd., London, GB; Class D25, AN 89-112334 XP002026108 & JP 01 060 693 A (AMANO PHARM KK) , 7 Mars 1989
- I.LAI, KUO-YANN: "liquid detergents", 1996, MARCEL DEKKER,

## Description

La présente invention concerne une composition pour le traitement d'objets que l'on souhaite nettoyer et/ou désinfecter tels que par exemple, des instruments médicaux, chirurgicaux et/ou endoscopiques.

Bien que plus particulièrement développée pour de telles applications, la présente invention pourra également être utilisée dans tous les domaines, notamment ménagers ou professionnels, dans lesquels on est amené à nettoyer et/ou désinfecter des objets, des surfaces ou autres.

Actuellement, lorsque l'on souhaite utiliser des objets susceptibles de présenter une contamination microbienne, il est connu de les traiter à l'aide de compositions désinfectantes.

Si lesdits objets présentent de plus des taches ou salissures, il est également nécessaire, tout d'abord, de les nettoyer. Pour cela, on connaît de nombreuses compositions détergentes.

Toutefois, dans la quasi totalité des cas, il est à noter que les micro-organismes présents sur les objets ainsi nettoyés n'ont pas été totalement éliminés.

En effet, malgré l'action desdites compositions, des composants organiques des taches ou salissures ne sont pas éliminés de façon efficace. Ainsi, les micro-organismes que ces taches ou salissures contiennent restent protégés notamment dans les résidus organiques non détruits et ne peuvent pas être atteints par les agents désinfectants.

En conséquence, de telles compositions ne sont pas entièrement satisfaisantes lorsqu'elles sont utilisées dans des environnements nécessitant une hygiène renforcée, telle que, notamment les environnements médicaux, hospitaliers ou autres.

Cela étant, il a déjà été proposé, dans le document FR-A-2.273.556, une composition à base d'halogénures d'ammonium quaternaire et de protéases. Toutefois les résultats d'une telle composition, notamment en terme de désinfection, peuvent être améliorés.

Le but de la présente invention est de proposer une composition pour le traitement d'objets qui pallie les inconvénients précités et permette à la fois de les nettoyer et/ou de les désinfecter de manière complète.

Un autre but de la présente invention est de proposer une composition pour le traitement d'objets, utilisant des enzymes, qui soit stable et permette ainsi d'éviter toute activité enzymatique avant son utilisation.

Un autre but de la présente invention est de proposer une composition pour le traitement d'objets dans laquelle l'utilisation d'enzymes soit compatible avec des agents désinfectants.

Un autre but de la présente invention est de proposer une composition pour le traitement d'objets que l'on souhaite nettoyer et/ou désinfecter qui ne présente pas d'action corrosive ni d'activité toxique.

Un autre but de la présente invention est de proposer une composition pour le traitement d'objets que l'on souhaite nettoyer et/ou désinfecter qui soit utilisable manuellement et/ou en machine.

Un autre but de la présente invention est de proposer une composition pour le traitement d'objets que l'on souhaite nettoyer et/ou désinfecter qui puisse être stockée sous forme de poudre.

Un autre but de la présente invention est de proposer une composition pour le traitement d'objets que l'on souhaite nettoyer et/ou désinfecter qui permette, après dilution, notamment dans de l'eau dure, d'obtenir une solution dont la limpidité soit conservée.

D'autres buts et avantages de la présente invention apparaîtront au cours de la description qui va suivre qui n'est donnée qu'à titre indicatif et qui n'a pas pour but de la limiter.

La présente invention concerne une composition pour le traitement d'objets tels que, par exemple, des instruments médicaux, chirurgicaux et/ou endoscopiques, comprenant, au moins, en association, des principes actifs anti-microbiens, constitués de sels d'ammonium quaternaire et/ou de dérivés de biguanides et/ou d'alkylaminoalkylamines et/ou de dérivés de phosphonium et/ou d'agents oxydants libérateurs d'oxygène, et des agents enzymatiques, constitués par un mélange contenant des enzymes encapsulés d'au moins deux familles différentes, aptes à nettoyer par action détergente lesdits objets et/ou à désinfecter, ainsi que de l'E.D.T.A.

Bien que plus particulièrement développée pour le traitement des instruments médicaux, chirurgicaux et/ou endoscopiques que l'on souhaite nettoyer et/ou désinfecter, il est à noter que la présente invention pourra également être utilisée dans tous les domaines, notamment ménagers et/ou professionnels, dans lesquels on souhaite traiter des objets, des surfaces ou autres.

Pour cela, lesdits principes actifs anti-microbiens sont constitués, de sels d'ammonium quaternaire et/ou de dérivés de biguanides et/ou d'alkylaminoalkylamines et/ou de dérivés de phosphonium et/ou d'agents oxydants libérateurs d'oxygène. Lesdits agents enzymatiques sont constitués d'un mélange contenant des enzymes d'au moins deux familles différentes.

Ce mélange permet d'éliminer l'ensemble des composants organiques susceptibles de former les taches ou salissures présents sur les objets traités. Il présente un effet synergique et assure ainsi la disparition rapide et complète des lipides, glucides et/ou protides entre autres constituant du biofilm.

L'efficacité des agents anti-microbiens est ainsi renforcée et ceci d'autant plus que les micro-organismes que l'on souhaite détruire sont généralement contenus au sein des composants formant les parties organiques des taches ou salissures.

Le choix des agents actifs retenus offre donc, simultanément un nettoyage et une désinfection des objets traités dont les résultats permettent leur utilisation dans des conditions d'hygiène optimales.

Ledit mélange enzymatique est constitué, par exemple, de lipages, d'amylases et de protéases.

La composition conforme à l'invention peut également contenir, éventuellement, d'autres enzymes aptes à intervenir dans un processus d'élimination des souillures organiques.

A ce sujet, les quantités d'enzymes utilisées forment, par exemple, en poids, 0,1 % à 25 % de la composition.

Il est à remarquer que l'on a obtenu de bons résultats en utilisant des enzymes encapsulés ce qui permet d'obtenir une composition stable en évitant toute activité enzymatique avant son utilisation.

Afin d'autoriser une bonne compatibilité des enzymes et des agents désinfectants et pour éviter ainsi la diminution de l'activité des enzymes et/ou des agents désinfectants, on choisira avantageusement une combinaison à caractère non oxydant.

Les agents anti-microbiens forment, par exemple, en poids, entre 0,1 % et 25 % de la composition. Ils sont choisis, notamment, en raison de leur niveau d'activité, de la largeur de leur spectre et/ou de leur absence de toxicité.

Selon un exemple particulier de mise en oeuvre de l'invention, la composition comprend également, si nécessaire, des agents aptes à renforcer son action détergente tel que, notamment, des tensio-actifs non-ioniques.

Il s'agit, par exemple, d'alcools gras éthoxylés et/ou d'alkylphénols éthoxylés et/ou de copolymères d'oxyde éthylène et/ou de propylène et/ou d'alkylpolyglycosides.

La composition conforme à l'invention se présente, par exemple, sous forme de poudre, notamment soluble en toute proportion dans l'eau.

A ce sujet, il est à noter que certains des agents anti-microbiens choisis ne sont initialement disponibles que sous forme liquide. Cela est le cas, par exemple, du chlorure d'alkyldecydiméthylammonium.

Afin d'éviter que la composition conforme à l'invention ne se présente en conséquence sous la forme d'une poudre trop humide contenant des agglomérats entraînant une hétérogénéité de la composition, on prévoit que ladite composition pourra comprendre, en outre, des réserves alcalines et/ou des agents complexants, aptes à absorber les parties liquides de manière à pouvoir obtenir une composition prête à l'emploi sous forme de poudre.

Il pourra s'agir, par exemple, de carbonate de soude, de sulfate de soude et/ou de tripolyphosphate. Il pourra également s'agir, entre autres, de dérivés phosphatés.

Cela étant, la composition conforme à l'invention devant éventuellement permettre, en solution dans l'eau, de nettoyer des objets tranchants et/ou piquants, susceptibles de blesser gravement la personne les manipulant, il est nécessaire dans ce cas de pouvoir assurer la limpidité de ladite solution.

Pour cela, ladite composition comprend, en outre, éventuellement, un complexant supplémentaire tel que celui connu sous le nom d'E.D.T.A.

Il est d'ailleurs à noter que le choix de ce composant présente l'avantage d'offrir un effet synergique, en combinaison avec les agents anti-microbiens utilisés, sur le plan de la désinfection, notamment vis-à-vis des bacilles à gram négatif tels que les pseudomonas, ces derniers constituant une des familles majeures à l'origine des infections nosocomiales.

La composition conforme à l'invention pourra également comprendre, notamment, des agents anti-corrosifs, par exemple actifs ou passifs.

Par ailleurs, la composition comprend en outre, éventuellement, un ou des tiers solvants, notamment de type alcools et/ou glycols.

On donne dans la suite un exemple de formulation, en pourcentage en poids, de la composition conforme à l'invention sous forme de poudre :

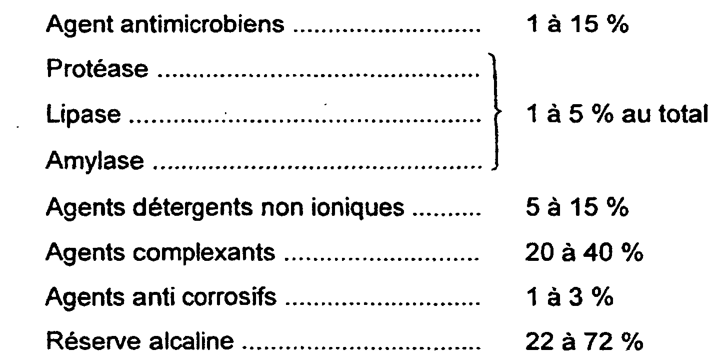

De manière avantageuse, les différents constituants de la composition conforme à l'invention sous forme de poudre et leurs proportions, en pourcentage en poids, pourront être choisis, par exemple, de la manière suivante :

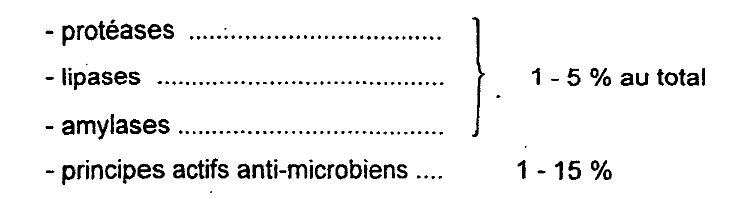

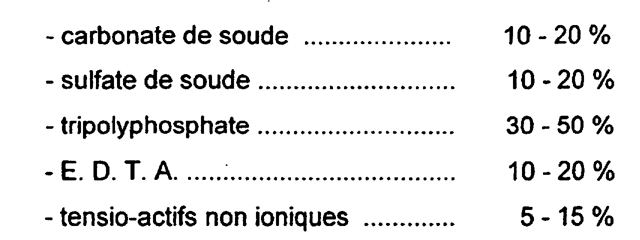

la somme des composants en poids étant égal à 100.

La composition conforme à l'invention pourra également, par exemple, être sous forme liquide. Un exemple de formulation correspondant, en pourcentage en poids, est donné dans la suite :

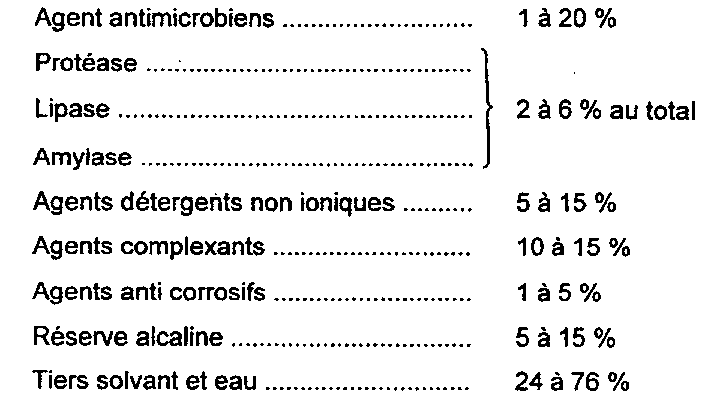

Par ailleurs, à titre d'exemple, le pH de la composition conforme à l'invention est fixé, sensiblement, entre 7 et 12 à la dilution d'emploi, cette dernière variant, en poids, par exemple de 0,1 % à 5 % par volume d'eau.

Naturellement, d'autres mises en oeuvre de la présente invention à la portée de l'homme de l'art auraient pu être envisagées sans pour autant sortir du cadre de la présente demande.

## Revendications

1. Composition pour le traitement d'objets tels que, par exemple, des instruments médicaux, chirurgicaux et/ou endoscopiques, comprenant, au moins, en association, des principes actifs anti-microbiens, constitués de sels d'ammonium quaternaire et/ou de dérivés de biguanides et/ou d'alkylaminoalkylamines, et/ou de dérivés de phosphonium et/ou d'agents oxydants libérateurs d'oxygène, et des agents enzymatiques, constitués par un mélange contenant des enzymes encapsulés d'au moins deux familles différentes, aptes à nettoyer par action détergente lesdits objets et/ou à désinfecter, ainsi que de l'E.D.T.A.

2. Composition selon la revendication 1, dans laquelle ledit mélange enzymatique est constitué de lipases, d'amylases et de protéases.

3. Composition selon la revendication 1 comprenant ou entre des agents aptes à renforcer l'action détergente.

4. Composition selon la revendication 3, dans laquelle les agents aptes à renforcer l'action détergente sont constitués par des alcools gras éthoxylés et/ou des alkylphénols éthoxylés et/ou des copolymères d'oxyde éthylène et/ou de propylène et/ou des alkylpolyglycosides.

5. Composition selon la revendication 1 comprenant en outre des agents complexants supplémentaires.

6. Composition selon la revendication 1 comprenant en outre des réserves alcalines.

7. Composition selon la revendication 1 comprenant en outre des agents anti-corrosifs actifs ou passifs.

8. Composition selon la revendication 1, présentant un pH sensiblement compris entre 7 et 12 à la dilution d'emploi.

9. Composition selon la revendication 1 comprenant un ou des tiers solvants de type alcools et/ou glycols.

10. Composition selon la revendication 1, se présentant sous la forme de poudre, comprenant, en pourcentage en poids : 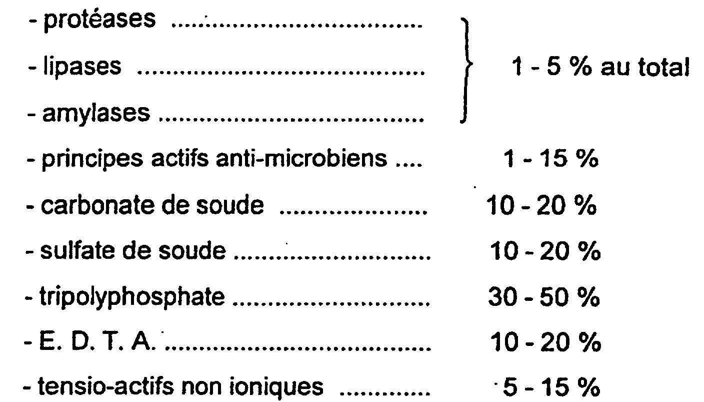 la somme des composants en poids étant égal à 100.

## Patentansprüche

1. Zusammensetzung zur Behandlung von Gegenständen, wie z.B. ärtzlichen, chirurgischen und/oder endoskopischen Instrumenten, umfassend wenigstens, in Verbindung, antimikrobielle Wirkstoffe, die aus quaternären Ammoniumsalzen und/oder Biguanidderivaten und/oder Alkylaminalkylaminen und/oder Phosphoniumderivaten und/oder Sauerstoffabgebenden Oxydationsmitteln bestehen, und enzymhaltige Agenzien, die aus einer Mischung bestehen, die eingekapselte Enzyme wenigstens zweier Familien enthält, welche geeignet sind, die genannten Gegenstände durch eine reinigende Wirkung zu reinigen und/oder zu entseuchen, sowie E.D.T.A.

2. Zusammensetzung nach Anspruch 1, bei der die enzymhaltige Mischung aus Lipasen, Amylasen und Proteasen besteht.

3. Zusammensetzung nach Anspruch 1, umfassend außerdem Agenzien, die geeignet sind, die reinigende Wirkung zu verstärken.

4. Zusammensetzung nach Anspruch 3, bei der die Agenzien, die geeignet sind, die reinigende Wirkung zu verstärken, aus ethoxylierten Fettalkoholen und/oder ethoxylierten Alkylphenolen und/oder Ethylenoxid-Copolymeren und/oder Propylen und/oder Alkylpolyglycosiden bestehen.

5. Zusammensetzung nach Anspruch 1, umfassend außerdem zusätzliche komplexierende Agenzien.

6. Zusammensetzung nach Anspruch 1, umfassend außerdem Alkalireserven.

7. Zusammensetzung nach Anspruch 1, umfassend außerdem aktive oder passive Korrosionsschutzmittel.

8. Zusammensetzung nach Anspruch 1, die einen pH-Wert aufweist, der bei Gebrauchsverdünnung im wesentlichen zwischen 7 und 12 liegt.

9. Zusammensetzung nach Anspruch 1, umfassend ein oder mehrere dritte Lösungsmittel der Art Alkoholen und/oder Glycolen.

10. Zusammensetzung nach Anspruch 1, die in der Form eines Pulvers ausgestaltet ist, umfassend, in Gewichtsprozent : 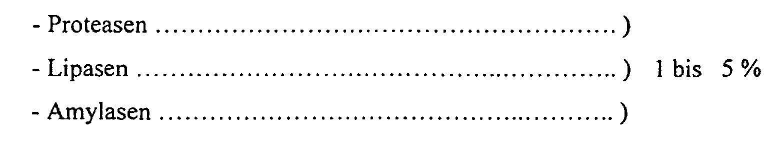 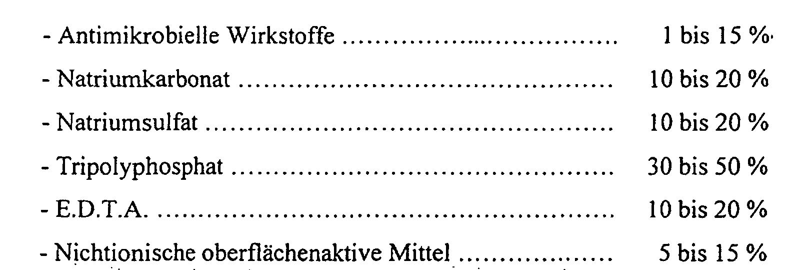 wobei die Summe der Bestandteile in Gewicht gleich 100 ist.

## Claims

1. A compound for the treatment of objects such as, for example, medical, surgical and/or endoscopic instruments, comprising at least, in association, antimicrobial active principles, consisting of quaternary ammonium salts and/or derivates of biguanides and/or alkylaminoalylamines, and/or phosphonium derivates and/or oxygen-releasing oxidising agents, containing encapsulated enzymes of at least two different families, capable of cleaning by a detergent effect said objects and disinfecting, as well as E.D.T.A.

2. A compound according to claim 1, wherein said enzymatic mixture consists of lipases, amylases and proteases.

3. A compound according to claim 1 comprising moreover agents capable of reinforcing the detergent action.

4. A compound according to claim 3, wherein the agents capable of reinforcing the detergent action consist of fat ethoxylated alcohols and/or ethoxylated alkylphenols and/or ethylene oxide and/or propylene copolymers and/or alkylpolyglycosides.

5. A compound according to claim 1 comprising moreover additional complexing agents.

6. A compound according to claim 1, comprising moreover alkaline reserves.

7. A compound according to claim 1, comprising moreover active or. passive anticorrosive agents.

8. A compound according to claim 1, whereof the pH ranges substantially between 7 and 12 at the dilution of usage.

9. A compound according to claim 1 comprising one or several alcohol and/or glycol-type solvent thirds.

10. A compound according to claim 1, in the form of powder, comprising in weight percentage:
| | |
|---|---|
| - proteases | |
| - lipases | 1 - 5 % in total |
| - Amylases | |
| - active antimicrobial principles | 1 - 15 % |
| - sodium carbonate | 10 - 20 % |
| - sodium sulphate | 10 - 20 % |
| - tripolyphosphate | 30 - 50 % |
| - E.D.T.A. | 10 - 20 % |
| - non ionic surfactants | 5 - 15 % |
the sum of the components in weight being equal to 100.
